# EUROPÄISCHE PATENTSCHRIFT

(11) **EP 1 027 221 B1**
(45) Veröffentlichungstag und Bekanntmachung des Hinweises auf die Patenterteilung: **19.12.2001**
(21) Anmeldenummer: 98958228.3
(22) Anmeldetag: 21.10.1998
(51) Int. Cl.: B41N 3/06

(54) **VERWENDUNG VON ALKYLPOLYGLYCOSIDEN IN DRUCKFARBENREINIGERN**
APPLICATION OF ALKYL POLYGLUCOSIDES IN PRINTING INK CLEANERS
UTILISATION D'ALKYLPOLYGLYCOSIDES DANS DES NETTOYEURS D'ENCRE D'IMPRIMERIE

(30) Priorität: 30.10.1997 DE 19747892
(43) Veröffentlichungstag der Anmeldung: 16.08.2000
(73) Patentinhaber: Cognis Deutschland GmbH, 40589 Düsseldorf (DE)
(72) Erfinder: FIES, Matthias, D-47800 Krefeld (DE)
(86) Internationale Anmeldenummer: EP9806690
(87) Internationale Veröffentlichungsnummer: WO9922943

(56) Entgegenhaltungen:
- EP-A- 0 498 545
- EP-A- 0 509 608
- DE-A- 3 903 465
- US-A- 5 616 548
- DATABASE WPI Section Ch, Week 9022 Derwent Publications Ltd., London, GB; Class E19, AN 90-167378 XP002092899 & JP 02 107492 A (IWATSU ELECTRIC KK) , 19. April 1990

## Beschreibung

### Gebiet der Erfindung

Die Erfindung betrifft die Verwendung von Alkylpolyglycosiden in Druckfarbenreinigern. Die Erfindung betrifft weiterhin ein Verfahren zur Entfernung von Druckfarben und/oder Ölrückständen, die sich auf der Oberfläche von Druckmaschinen, Druckplatten und dergleichen sowie der dazugehörigen Ausrüstung bilden, wobei man die Oberfläche mit einem Reinigungsmittel behandelt. die mindestens ein Alkylpolyglycosid enthält.

### Stand der Technik

**EP 498 545 B1** beschreibt ein Verfahren zur Entfernung von Ölrückständen, der sich auf der Oberfläche von Druckmaschinen. Druckplatten und der dazugehörigen Ausrüstung bilden, das folgenden Schritt umfaßt: Behandeln der Oberfläche mit einem Reinigungsmittel, das mindestens einen C₆- oder höheren Alkylester einer Fettsäure enthält. Neben diesen Fettsäureestern, die als Hauptbestandteil des Reinigungsmittels fungieren, können andere komplementäre Bestandteile im Reinigungsmittel enthalten sein, beispielsweise Tenside, Emulgatoren, Pflanzenöle, Fettsäure-C₁₋₅-Alkylester, Wasser, organische Lösungsmittel, Säuren, Basen, Alkalien, Puffersysteme. Sequestriermittel und Korrosionsinhibitoren.

**WO 94/17143** beschreibt Reinigungs-Zusammensetzungen zum Entfernen unerwünschter Farbe und ähnlicher "Beschichtungen" von einem Substrat, wobei diese Mittel 5-Ring-Lactame sowie Triglyceride von Monocarbonsäuren mit 1 bis 4 C-Atomen enthalten.

**WO 90/03419** beschreibt die Verwendung von C₁₋₅-Alkylestern von aliphatischen C₈₋₂₂-Monocarbonsäuren zum Entfernen von Fett. Tinte und dergleichen von Druckmaschinen, insbesondere Offset-Druckmaschinen. Die genannten Ester werden vorzugsweise in Abmischung mit Pflanzenölen und einem Tensid eingesetzt. Das Tensid wird dabei vorzugsweise ausgewählt aus der Klasse der Polyglykolether aliphatischer C₈₋₂₂-Alkohole, wobei Anlagerungsprodukte von 7 bis 14 Mol Ethylenoxid pro Mol eines C₁₂₋₂₂-Fettalkohols ganz besonders bevorzugt sind.

### Beschreibung der Erfindung

Aufgabe der vorliegenden Erfindung war es, neue effektive Druckfarbenreiniger bereitzustellen. Insbesondere sollte ein Verfahren zur Entfernung von Druckfarben und/oder Ölrückständen, die sich auf der Oberfläche von Druckmaschinen, Druckplatten und dergleichen sowie der dazugehörigen Ausrüstung bilden, bereitgestellt werden.

Überraschenderweise wurde gefunden, daß Alkylpolyglycoside, die zur Klasse der nichtionischen Tenside zählen, alleine oder in Kombination mit anderen Substanzen sich dazu eignen, Druckfarben nachhaltig von festen Oberflächen zu entfernen. Insbesondere eignen sich die genannten Alkylpolyglycoside zur Entfernung von Druckfarben und/oder Ölrückständen, die sich auf der Oberfläche von Druckmaschinen, Druckplatten und dergleichen sowie der dazugehörigen Ausrüstung bilden.

Der Begriff der "festen Oberfläche" wird im Rahmen der vorliegenden Erfindung in dem Sinne gebraucht, daß hierunter ausschließlich solche Anwendungen fallen. bei denen die zu reinigende Oberfläche lediglich von unerwünschten darauf befindlichen Ablagerungen wie Druckfarben, Druckfarbenrückständen, Ölrückständen und dergleichen befreit wird, die Oberfläche an sich bei diesem Vorgang jedoch nicht zerstört wird. Nicht in den Rahmen der vorliegenden Erfindung fallen dementsprechend solche Anwendungen, bei denen die Entfernung von Druckfarben und dergleichen mit einer substantiellen Zerstörung bzw. einem substantiellen Abbau der Oberfläche selbst einhergeht, wie es beispielsweise bei der Entfernung von Druckfarben aus bedruckten Altpapieren der Fall ist. Bei diesem letztgenannten Prozeß handelt es sich darum. daß das eingesetzt Papier als solches nicht erhalten bleibt, sondern nach dem Prozeß in Form vom Fragmenten (Papierfaserbrei) vorliegt.

Gegenstand der vorliegenden Erfindung ist die Verwendung von Alkylpolyglycosiden (I)

R (G)ₓ (I)

worin R ein linearer, gesättigter Alkylrest mit 8 bis 22 C-Atomen und (G)ₓ ein Glycosid- oder Oligoglycosid-Rest mit einem Oligomerisationsgrad x von 1 bis 10 ist, in Druckfarbenreinigern.

Die **Alkylglycoside (I)** sind seit langem bekannte oberflächenaktive Stoffe, die aus Zuckern und aliphatischen, primären Alkoholen mit 8 bis 22 C-Atomen unter Acetalisierung herstellbar sind. Als Zuckerkomponente (Glycosen) kommen bevorzugt Glucose, daneben aber auch Fructose, Mannose, Galactose, Telose, Gulose. Allose, Altrose, Idose, Arabinose, Xylose, Lyxose, Libose und Gemische davon in Frage.

Bevorzugt wegen der leichten Zugänglichkeit und der guten Anwendungseigenschaften sind die Acetalisierungsprodukte der Glucose mit Fettalkoholen, die beispielsweise aus natürlichen Fetten und Ölen nach bekannten Verfahren erhältlich sind, insbesondere mit linearen, primären, gesättigten und ungesättigten Fettalkoholen mit 8 bis 22 C-Atomen.

Bezüglich des Glycosidrestes (G)ₓ gilt, daß sowohl Monoglycoside (x = 1), bei denen ein Zuckerrest glycosidisch mit dem Fettalkohol verbunden ist, als auch oligomere Glycoside mit einem Oligomerisationsgrad x = 2 bis 10 geeignet sind. In der Regel liegen Gemische von Mono- und Oligoglycosiden vor.

Bevorzugt geeignet sind solche Alkylglycoside (I), in welcher R eine Alkylgruppe mit 8 bis 22 C-Atomen und (G)ₓ ein Glycosid- oder Oligoglycosidrest mit einem Oligomerisationsgrad x = 1 bis 10 ist. Ganz besonders bevorzugt ist R dabei eine Alkylgruppe mit 8 bis 14 C-Atomen. Der mittlere Oligomerisationsgrad liegt vorzugsweise im Bereich von 1 bis 1.5.

In vielen Fällen kann das Ausmaß der Entfernung von Druckfarben von festen Oberflächen gesteigert werden, wenn die erfindungsgemäßen Alkylpolyglycoside in Kombination mit Fettsäureestern und/oder einem oder mehreren Tensiden eingesetzt werden. Die Tenside können dabei aus den Klassen der anionischen Tenside (außer Alkylpolyglykosiden (I), die ja ohnehin obligatorischer Bestandteil der erfindungsgemäß einzusetzenden Verbindungen sind) gewählt werden. Dabei unterliegt die Auswahl des Tensids keinerlei Beschränkungen, das heißt es lassen sich an sich alle dem Fachmann einschlägig bekannten anionischen, kationischen und nichtionischen Tenside einsetzen. Das Gewichtsverhältnis der Verbindungen (I) zu den genannten fakultativen Bestandteilen liegt dabei im Bereich von 1 : 20 bis 20 : 1.

In Gegenwart der erfindungsgemäß einzusetzenden Alkylpolyglycoside (I) lassen sich Druckfarben und/oder Ölrückstände, beispielsweise Zeitungsrotationsfarben. Buchdruckfarben, Offsetdruckfarben, Illustrationstiefdruckfarben, Flexodruckfarben, Laserdruckfarben und/oder Verpackungstiefdruckfarben von festen Oberflächen von Druckmaschinen, Druckplatten und dergleichen entfernen.

Ein weiterer Gegenstand der Erfindung ist ein Verfahren zur Entfernung von Druckfarben und/oder Ölrückständen, die sich auf der Oberfläche von Druckmaschinen, Druckplatten und dergleichen sowie der dazugehörigen Ausrüstung bilden, wobei man die Oberfläche mit einem Reinigungsmittel behandelt, das mindestens ein Alkylpolyglycosid (I)

R (G)ₓ (I)

worin R ein linearer, gesättigter Alkylrest mit 8 bis 22 C-Atomen und (G)ₓ ein Glycosid- oder Oligoglycosid-Rest mit einem Oligomerisationsgrad x von 1 bis 10 ist, enthält.

In einer Ausführungsform dieses Verfahrens setzt man die Alkylpolyglykoside (I) in Kombination mit Fettsäureestern (II) der Struktur

R¹-CO₂-R² (II)

worin R¹ einen gesättigten oder ungesättigten, geradkettigen oder verzweigten Alkylrest mit 6 bis 23 C-Atomen und der Rest R2 einen gesättigten oder ungesättigten Alkylrest mit 1 bis 24 C-Atomen bedeuten, und/oder einen oder mehreren Tensiden ein.

Die nachfolgenden Beispiele dienen der Erläuterung der Erfindung und sind nicht einschränkend zu verstehen.

### Beispiele

### Verwendete Substanzen

| | |
|---|---|
| Glucopon 650 EC | C₈₋₁₄-Alkylpolyglucosid mit Oligomerisationsgrad x = 1.5; 50%-ige wäßrige Lösung (Fa. Henkel KgaA, Düsseldorf) |
| Glucopon 225 CSUP | C₈₋₁₀-Alkylpolyglucosid mit Oligomerisationsgrad x = 1,6; 50%-ige wäßrige Lösung (Fa. Henkel KgaA. Düsseldorf) |
| Glucopon 600 EC | C₈₋₁₄-Alkylpolyglucosid mit Oligomerisationsgrad x = 1,4; 50%-ige wäßrige Lösung (Fa. Henkel KgaA, Düsseldorf) |

Texaprint SRM: Rapsfettssäuremethylester (Fa. Henkel KGaA. Düsseldorf)
Texaprint SKEH: Kokosfettsäure-2-Ethylhexylester (Fa. Henkel KGaA, Düsseldorf)
Texaprint SLIB: Laurinsäureisobutylester (Fa. Henkel KGaA, Düsseldorf)
Texaprint AC 3160: Korrosionsschutzmittel (Fa. Henkel KGaA. Düsseldorf)
Disponil TL 55: nichtionisches Tenside (Fa. Henkel KGaA, Düsseldorf)
Dehydol LS 57: nichtionisches Tensid (Fa. Henkel KGaA. Düsseldorf)

Die Mengenangaben in den nachfolgenden Beispielen sind Gewichtsteile.

### Beispiel 1

50 Teile TEXAPRINT SRM
40 Teile Wasser
3 Teile TEXAPRINT AC 3160
2 Teile Disponil TL 55
5 Teile Glucopon 650 EC

### Beispiel 2

50 Teile TEXAPRINT SKEH
40 Teile Wasser
3 Teile TEXAPRINT AC 3160
5 Teile Glucopon 215 CSUP
2 Teile Dehydol LS 7

### Beispiel 3

50 Teile TEXAPRINT SLIB
42 Teile Wasser
3 Teile TEXAPRINT AC 3160
5Teile Glucopon 600 EC

### Anwendungsbeispiel

Auf einer Heidelberg-Offset-Druckmaschine wurden mehrere tausend Papierbögengedruckt. Anschließend wurden die Walzen mit den Reinigerformulierungen gemäß den Beispielen 1 bis 3 gewaschen. Die Druckfarbe ließ sich rasch und problemlos entfernen, die Reiniger-Emulsion ließ sich rückstandsfrei von den Walzen abreiben.

## Patentansprüche

1. Verwendung von Alkylpolyglycosiden der allgemeinen Formel (I)
R (G)ₓ (I)
worin R ein linearer, gesättigter Alkylrest mit 8 bis 22 C-Atomen und (G)ₓ ein Glycosid- oder Oligoglycosid-Rest mit einem Oligomerisationsgrad x von 1 bis 10 ist, in Druckfarbenreinigern.

2. Verwendung nach Anspruch 1, wobei R eine Alkylgruppe mit 8 - 14 C-Atomen ist.

3. Verwendung nach Anspruch 1 oder 2, wobei x im Bereich von 1 bis 1,5 liegt.

4. Verwendung nach einem der Ansprüche 1 bis 3, wobei G ein Glucosidrest ist.

5. Verfahren zur Entfernung von Druckfarben und/oder Ölrückständen, die sich auf der Oberfläche von Druckmaschinen, Druckplatten und dergleichen sowie der dazugehörigen Ausrüstung bilden, wobei man die Oberfläche mit einem Reinigungsmittel behandelt, das mindestens ein Alkylpolyglycosid (I)
R (G)ₓ (I)
worin R ein linearer, gesättigter Alkylrest mit 8 bis 22 C-Atomen und (G)ₓ ein Glycosid- oder Oligoglycosid-Rest mit einem Oligomerisationsgrad x von 1 bis 10 ist. enthält.

6. Verfahren nach Anspruch 5, wobei man die Alkylpolyglykoside (I) in Kombination mit Fettsäureestern (II) der Struktur
R¹-CO₂-R² (II)
worin R¹ einen gesättigten oder ungesättigten, geradkettigen oder verzweigten Alkylrest mit 6 bis 23 C-Atomen und der Rest R² einen gesättigten oder ungesättigten Alkylrest mit 1 bis 24 C-Atomen bedeuten, und/oder einem oder mehreren Tensiden einsetzt.

## Claims

1. The use of alkyl polyglycosides of the general formula (I)
R(G)ₓ (I)
where R is a linear saturated alkyl radical having 8 to 22 carbon atoms and (G)ₓ is a glycoside or oligoglycoside radical having a degree of oligomerization, x, of from 1 to 10 in printing ink cleaners.

2. The use as claimed in claim 1, where R is an alkyl group having 8 - 14 carbon atoms.

3. The use as claimed in claim 1 or 2, wherein x is within the range from 1 to 1.5.

4. The use as claimed in any of claims 1 to 3, where G is a glucoside radical.

5. A method of removing printing inks and/or oil residues which form on the surface of printing machines, printing plates and the like and also the associated equipment, which comprises treating said surface with a cleaning composition comprising at least one alkyl polyglycoside (I)
R(G)ₓ (I)
where R is a linear saturated alkyl radical having 8 to 22 carbon atoms and (G)ₓ is a glycoside or oligoglycoside radical having a degree of oligomerization, x, of from 1 to 10.

6. The method as claimed in claim 5, wherein the alkyl polyglycosides (I) are used in combination with fatty acid esters (II) of the structure
R¹-CO₂-R² (II)
where R¹ is a saturated or unsaturated straight-chain or branched alkyl radical having 6 to 23 carbon atoms and the radical R² is a saturated or unsaturated alkyl radical having 1 to 24 carbon atoms and/or with one or more surfactants.

## Revendications

1. Utilisation d'alkylpolyglycosides de formule générale (I)
R(G)ₓ (I)
dans laquelle R représente un radical alkyle saturé linéaire comportant de 8 à 22 atomes de carbone et (G)ₓ un radical glycoside ou oligoglycoside ayant un degré d'oligomérisation x de 1 à 10, dans des produits de nettoyage d'encres d'imprimerie.

2. Utilisation selon la revendication 1,
dans laquelle
R est un groupe alkyle comportant de 8 à 14 atomes de carbone.

3. Utilisation selon la revendication 1 ou 2 dans laquelle x se situe dans un intervalle allant de 1 à 1,5.

4. Utilisation selon l'une des revendications 1 à 3,
dans laquelle
G est un radical glucoside.

5. Procédé d'élimination des encres d'imprimerie et/ou des résidus huileux qui se forment à la surface des machines d'imprimerie, des plaques d'imprimerie et analogues ainsi que de l'équipement correspondant,
dans lequel
on traite la surface avec un produit de nettoyage qui contient au moins un alkylpolyglycoside (I)
R(G)ₓ (I)
dans laquelle R représente un radical alkyle saturé linéaire comportant de 8 à 22 atomes de carbone et (G)ₓ un radical glycoside ou oligoglycoside ayant un degré d'oligomérisation x de 1 à 10.

6. Procédé selon la revendication 5,
dans lequel
on utilise les alkylpolyglycosides (I) en combinaison avec des esters d'acides gras (II) de structure
R¹-CO₂-R² (II)
dans laquelle R¹ représente un radical alkyle saturé ou insaturé, à chaîne droite ou ramifiée et, comportant de 6 à 23 atomes de carbone, et le radical R² représente un radical alkyle saturé ou insaturé comportant de 1 à 24 atomes de carbone, et/ou un ou plusieurs agents tensioactifs.
